(19) Europäisches Patentamt
European Patent Office
Office européen des brevets

(11) Publication number : 0 472 338 A2

(12) **EUROPEAN PATENT APPLICATION**

(21) Application number : 91307409.2

(51) Int. Cl.$^5$ : C07D 307/935

(22) Date of filing : 12.08.91

(30) Priority : 21.08.90 JP 221816/90

(43) Date of publication of application :
26.02.92 Bulletin 92/09

(84) Designated Contracting States :
AT BE CH DE DK ES FR GB GR IT LI LU NL SE

(71) Applicant : Kabushiki Kaisha Ueno Seiyaku Oyo Kenkyujo
4-8, 2-chome, Koraibashi Chuo-ku
Osaka-shi Osaka-fu (JP)

(72) Inventor : Ueno, Ryuji
7-29, Misaku-cho
Nishinomiya-shi, Hyogo-ken (JP)
Inventor : Oda, Tomio
B-203, Ueno Seiyaku Sanda Shikou-Ryo
1-29, Suzukakedai, Sanda-shi, Hyogo-ken (JP)

(74) Representative : Atkinson, Peter Birch et al
Marks & Clerk Suite 301 Sunlight House Quay Street
Manchester M3 3JY (GB)

(54) **Method of manufacturing prostaglandin intermediate.**

(57) The process according to the present invention can produce the ketolactones (3), important intermediates for the symthesis of 20-ethyl-prostanglandins, in remarkably improved yields and for a shortened period of time by reactiong the Corey aldehydes (2) with di-lower-alkyl (2-oxononyl)phosphates in halogenated hydrocarbons under the presence of lithium halides and tertiary amines, whereby said Corey aldehydes (2) are prepared through DMSO oxidation of the Corey lactones (1) in halogenated hydrocarbons.

EP 0 472 338 A2

BACKGROUND OF THE INVENTION

The present invention relates to a process of (1S, 5R, 6R, 7R)-6-[(E)-3-OXO-1-decanyl]-7-(4-aroyloxy)-2-oxabicyclo[3,3,0]octa-3-one(3) (hereinafter referred briefly to keto-lactons(3)) from the Corey lactones in a synthetic process of 20-ethyl-prostaglandins.

During the studies on various kinds of prostaglandin derivatives, it has been found that many of prostaglandins having the 10-chain elongated derived from the prostanoic acid skeleton represented by the following formula:

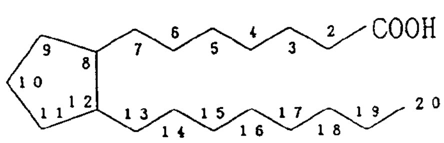

(α-chain)

(ω-chain)

especially derivatives (20-ethyl-prostaglandins) of them having 10 of a number of carbon atom in the ω-chain, exhibit characteristic phisiological activities.

The Corey method has been known for a long time and is currently used as a typical synthetic procedure of prostaglandins.

The synthesis shown in the following scheme is included in the Corey method, wherein the ketolactones (3) are obtained from the Corey lactones (1) via Corey aldehydes (2) as the intermediates.

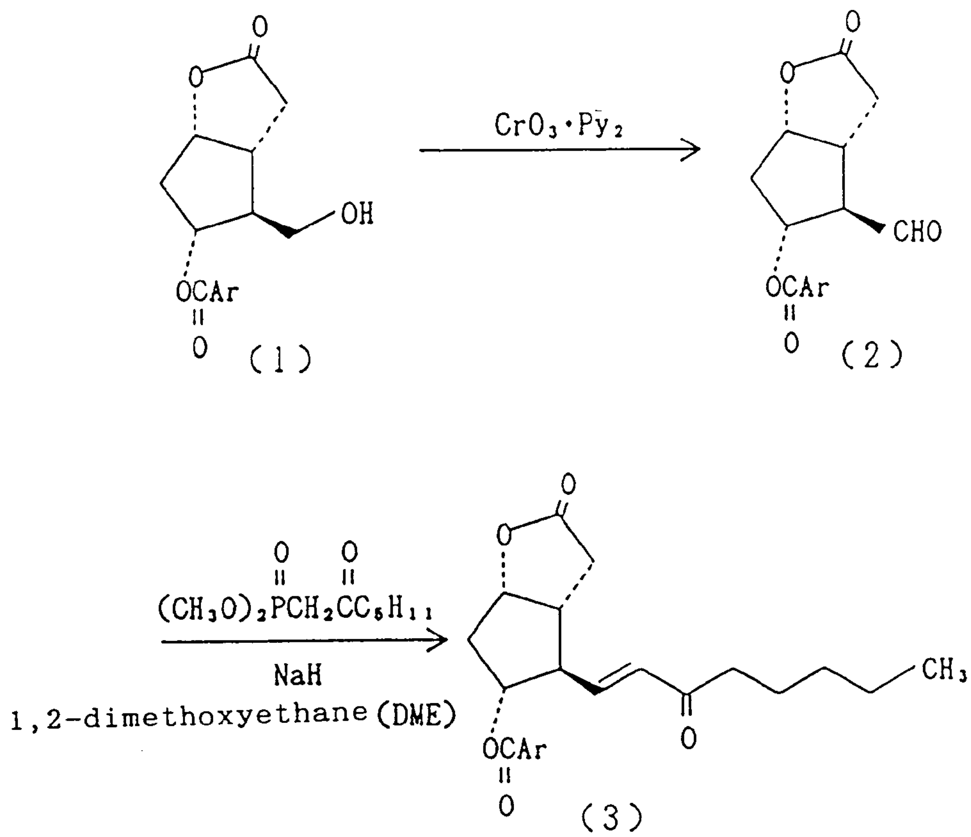


(1) (2) (3)

Corey lactones (1) are subjected to an oxidation reaction using pyridine/chromium trioxide complex (so called Collins oxidation) to form Corey aldehydes (2). The products (2) are allowed to react with an anion generated from the reaction of dimethyl (2-oxoalkyl)phosphonate with sodium hydride, thereby obtaining ketolactones (3). Solvents such as 1,2-dimethoxyethane (DME) or THF are used in the reaction to synthesize (3) from (2).

Yields in the above-described reactions are, however, from 50 to 60 % at best even in the reactions using the typical prostaglandin intermediate with the carbon atom number of 8 in ω-chain skeleton; the yields being more reduced for 20-ethyl-prostaglandin intermediates bearing the ω-side chain consisted of 10 carbon atoms.

SUMMARY OF THE INVENTION

The object of the present invention is to provide a process of the preparation of the ketolactones represented by the following formula (3):

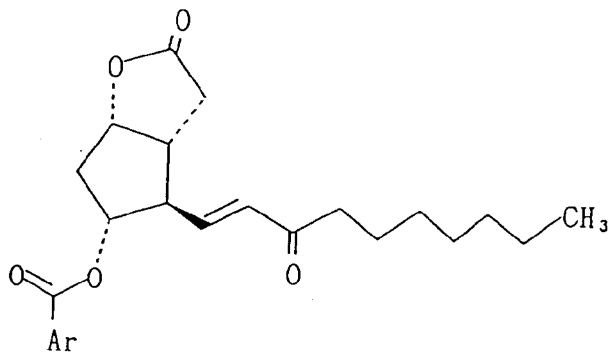

(3)

which are important compounds for the production of 20-ethyl-prostaglandins.

In the process of the present invention, the ketolactones are obtained from the reactions between the Corey aldehydes (2) and the phosphonate in a halogenated hydrocarbon, as a solvent, with a dipole moment of 0.9 D to 1.3 D in the presence of a lithium halide and a tertiary amine, and the Corey aldehydes (2) may be prepared by subjecting the Corey lactones (1) to DMSO oxidation in the aforementioned halogenated hydrocarbons, by which the yield of the ketolactones (3) can be extremely improved, and the reaction time for the preparation thereof can be shortened.

DETAILED DESCRIPTION OF THE INVENTION

The present invention relates to a process of preparing ketolactones (3);

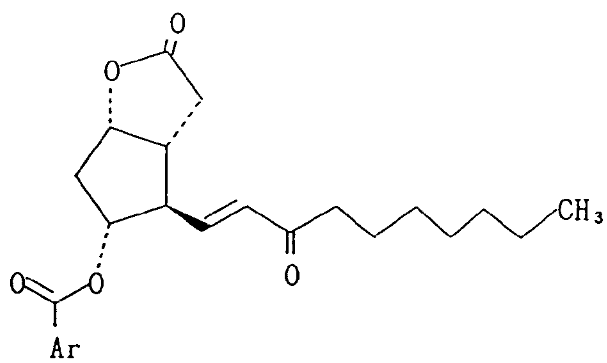

(3)

[wherein Ar represents an aromatic group,]
which comprises reacting Corey aldehydes (2):

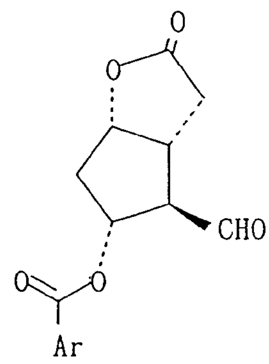

(2)

[wherein Ar repreents an aromatic group] with di-lower-alkyl (2-oxononyl)phosphonate:

$(RO)_2POCH_2CO(CH_2)_6CH_3$

[wherein R represents an alkyl group having a carbon atom number of 1 to 4] in which the reactions are carried out, in the presence of a lithium halide and a tertiary amine in a halogenated hydrocarbon, as a reaction solvent, with a dipole moment of 0.9 D to 1.3 D.

The present invention also relates to a process of preparing ketolactones (3), wherein the Corey aldehydes (2) are obtained by DMSO oxidation of the Corey lactones (1) of the following formula:

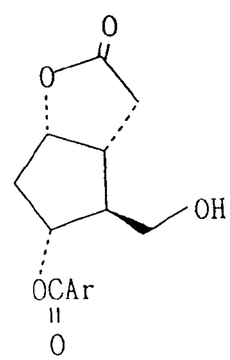

(1)

[wherein Ar is defined as above]
in the same solvents as are used in the synthesis of the ketolactones (3), followed by a reaction between the Corey aldehydes (2) and di-lower-alkyl (2-oxononyl)phosphonate using the same process as aforementioned. Protective group Ar represents an aromatic group such as phenyl, biphenyl, naphthyl groups and the like; phenyl and biphenyl groups are particularly preferable.

The reaction to obtain the Corey aldehydes (2) from the Corey lactones (1) is achieved by an oxidation reaction using dimethylsulfoxide (the oxidation reaction hereinafter referred to briefly as "DMSO oxidation"). Examples of the DMSO oxidation include so-called Pfilzner-Moffatt oxidation using DMSO, dicyclohexylcarbodiimide, trichloroacetic acid and pyridine, Swern oxidation using DMSO, oxyalylchloride and triethylamine, and Parikh-Doering oxidation using DMSO, sulfur trioxide-pyridine complex and triethylamine. Since the aldehydes produced by the DMSO oxidation are not susceptible any more to subsequent oxidation to the corresponding carboxilic group, the reaction is easily controlled to oxidize alcohols to the corresponding aldehydes in high yields. As aforementioned, the same solvents as used in the reaction process of the Corey aldehydes (2) to the ketolactones (3) can be used in the oxidation process, thereby permitting both reaction steps to proceed smoothly. The second step is allowed to proceed without removing the solvent used in the first reaction one, resulting in remarkably improved productivity and yield.

The amount of the solvent used in the oxidation reaction may be 5 to 20 ml per 1 g of the Corey lactone with the reaction temperature being -5 to 30 °C and the reaction time ranging from 3 to 5 hours.

The reaction for producing the ketolacton (3) from the Corey aldehyde(2) is carried out in the presence of a lithium halide and a tertiary amine. Included among halides are fluoride, chloride, bromide and iodide, and bromide is particularly preferable. Examples of the tertiary amine used are lower alkylamines such as trimethylamine, triethylamine and the like; heterocyclic amines such as 1,8-diazabicyclo-[5.4.0]unde-7-cene and 1,5-diazabicyclo[4.3.0]none-5-ene, and the like; and aromatic amines such as dimethylaniline, diethylaniline and the like. Triethylamine is particularly preferable.

Lithium halide and a tertiary amine are both preferably used at the ratios of one to three equivalents per one equivalent of the Cory aldehyde (2).

As is described above, halogenated hydrocarbons with a dipole moment in the range of 0.9 to 1.3 are suitable as the reaction solvent, dichloromethane being particularly preferred. When the two reaction steps, the first being DMSO oxidation of the Corey lactones (1) to the Corey aldehydes (2) and the second being introduction of the ω-chain into the Corey aldehydes (2) to form ketolactones (3), are carried out without isolation of the Corey aldehydes (2), the reaction of the second process is feared to be obstructed by the products of the first step in some manner, resulting in the decreased yield of the final product. In the present invention, however, the yields are, exceedingly high throughout these two steps.

The volume of the reactin solvent used is 5 to 50 ml per 1 g of the Corey aldehyde (2), particularly preferably 15 to 25 ml. The reaction temperature is in the range of -20 to 40 °C, particularly preferably 0 to 20 °C.

The alkyl phosphonate used in the present invention is di-lower-alkyl (2-oxononyl)phosphonates. Examples of the lower alkyl groups are those having a carbon atom number of 1 to 4, and methyl and ethyl are particularly preferable.

According to the present invention, the ketolactones (3) can be obtained using a di-lower-alkyl (2-oxononyl) phosphonate in high yields. The yields can reach up to 95 % or more, and the reaction can be completed within a length of time as short as 30 min. to 1.5 hours.

The present invention is illustrated below with reference to examples.

Example

Synthesis of (1S, 5R, 6R, 7R)-6-[(E)-3-oxo-1-decenyl]-7-(4-phenylbenzoyloxy)-2-oxabicyclo[3.3.0]octa-3-one(3)(α,β-unsaturated ketone (3)):

A magnetic stirrer was placed in a four-necked flask of 500 ml volume equipped with a thermometer, a gas-inlet and an outlet, the gas inlet being connected to an argon cylinder while the gas outlet connected to a bubbler.

A dichloromethane (100 ml) solution of Corey lactone (1) (40 g) was introduced in this reaction vessel and the solution was ice-cooled. After the internal temperature reached below 15 °C, dicyclohexylcarbodiimide (DCC; 23.5 g) was poured into the flask and then DMSO (60.5 ml) was added, followed by the addition of pyridine (2.3 ml). When the internal temperature reached 10 °C or less, trifluoroacetic acid (1.1 ml) was added and the solution was stirred for 3 hours at room temperature. After the mixed solution was ice-cooled again to 8 °C or less of the internal temperature, a 25 % aqueous solution of sodium hydrogensulfate was added to the solution and the reaction mixture was stirred for 15 min., followed by filtration.

After adding water (50ml) to the filtrate followed by stirring, the mixed solution was separated into the aqueous and organic layers. The organic layer was washed with aqueous sodium hydrogencarbonate and then with aqueous sodium chloride, followed by drying. After filtration, the filtrate was concentrated to give the Corey aldehyde (2).

During the procedure described above, dimethyl 2-oxononylphosphonate anion was generated separately as is described below.

A dichloromethane solution (100 ml) of 2-oxononyl-phosphonate (8.5 g) was poured into a 500 ml four-necked flask as described in the above. Lithium bromide monohydrate (5.3 g) was added to the solution and the solution was stirred for 15 min. at room temperature. After cooling the solution with ice at 5 °C or less, triethylamine (7.1 ml) was added to the reaction mixture and the solution was stirred for 1 hour.

A solution of the Corey aldehyde (2) in dichloromethane (75 ml) was poured into the above-described solution of dimethyl 2-oxononylphosphonate anion under ice-cooling and the resulting solution was stirred for 1.5 hours, followed by the addition of acetic acid (3 ml). The reaction product was treated in the usual work-up and the α,β-unsaturated ketone (3) was obtained. The crude product was chromatographed on a column of 200 g of silica gel with ethyl acetate/n-hexane (2/1) as an eluting solvent. Yield; 13.2 g, 96 % (through two reaction steps).

Comparative example 1

Dried pyridine (413 ml) was added to methylenechloride (4 l), and chromic anhydride (255 g) was added, as divided in three portins, to the mixed solution successively. After the reaction mixture was stirred for 2 hours at a temparature of below 26 °C, cellite (600 g) was added, followed by stirring for 15 min. A solution of the Corey lactone (1) (100 g) was added dropwise to the suspension cooled at 0 °C. After stirring for 30 min., sodium hydrogensulfate (776 g) was added slowly to the reaction mixture. After 10 min., the mixture was filtered using magnesium sulfate (750 g). Dimethyl (2-oxononyl)phosphonate anion generated with dimethyl(2-oxononyl)phosphonate (85.2 g) and sodium hydride (13.6 g) in dichloromethane was added to the filtrate and, after stirring for 1 hour at 0 °C, acetic acid (24 ml) was added to the solution. The crude product obtained after the usual work-up was chromatographed on a column of silica gel to yield the α,β-unsaturated ketone (3). Yield; 71.8 g, 53.3% (through two reactin steps).

Comparative Example 2

Into a 300 ml flask a solution of dimethyl-2-oxoheptylphosphonate (4.86 g) in dichloromethane (64.0 g) was introduced, lithium bromide mono hydrate (2.29 g) was added to the solution and the solution was stirred for 15 min. at room temperature. After cooling the solution with ice at 5 °C or less, triethylamine (3.05 ml) was added to the reaction mixture and the mixed solution was stirred for one hour.

A solution of the Corey aldehyde (2) which was prepared according to the same method as in the Example (5.01 g) in dichloromethane (31.3 ml) was poured into the above mixed solution with ice-cooling, and the solution obtained was stirred for one hour, followed by the addition of acetic acid (1.25 ml). The reaction product was treated in the usual work-up and the α,β-unsaturated ketone (3) was obtained. The crude product was choromatographed on a column of 230 g of silica gel with ethyl acetate/n-hexane (1/1) as an eluting slovent. Yield: 5.24 g, 77.4 %

Comparative Example 3

Into a 300 ml flask a solution of dimethyl-2-oxoheptylphosphonate (4.46 g) in dichloromethane (64.0 g) was introduced, lithium bromide unhydride (1.28 g) was added to the solution and the solution was stirred for 15 min. at room temparature. After cooling the solution with ice at 5 °C or less, triethylamine (4.19 ml) was added to the reaction mixture and the mixed solution was stirred for one hour.

A solution of the Corey aldehyde (2) which was prepared according to the same method as in the Example (5.01 g) in dicholoromethane (8.0 ml) was poured into the above mixed solution with ice-cooling, and the solution obtained was stirred for one hour, followed by the addition of acetic acid (1.72 ml). The reaction product was treated in the usual work-up and the α,β-unsaturated ketone (3) was obtained. The crude product was chromatographed on a column of 210 g of silica gel with ethyl acetate/n-hexane (1/1) as an eluting solvent. Yield: 5.59 g, 82.6 %

**Claims**

1. A process of preparing ketolactones (3) of the formula:

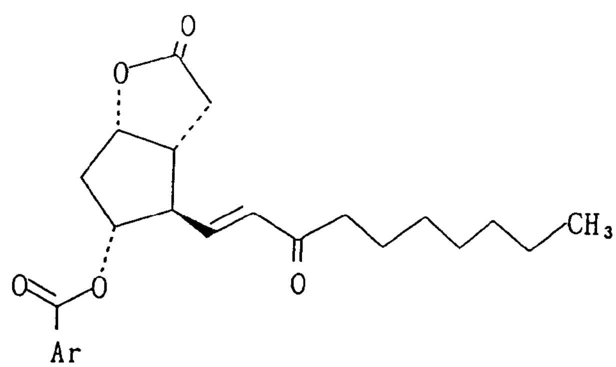

(3)

[wherein Ar represents an aromatic group]
which comprises reacting a Corey aldehyde (2):

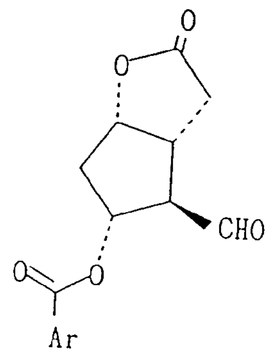

(2)

[wherein Ar represents an aromatic group] with a di-lower alkyl(2-oxononyl)phosphonate:

$$(RO)_2POCH_2CO(CH_2)_6CH_3$$

[wherein R represents a $C_1$ - $C_4$ alkyl group], in which the reaction is carried out in the presence of a lithium halide and a tertiary amine in a halogenated hydrocarbon as a solvent with a dipole moment in the range of 0.9 D to 1.3 D.

2. A preparation process according to Claim 1, wherein Ar is phenyl or biphenyl and the reaction solvent is dichloromethane.

3. A preparation process according to Claim 1, wherein the di-lower-alkyl (2-oxononyl)phosphonate is dimethyl (2-oxononyl)phosphonate.

4. A process of preparing of a ketolactone (3):

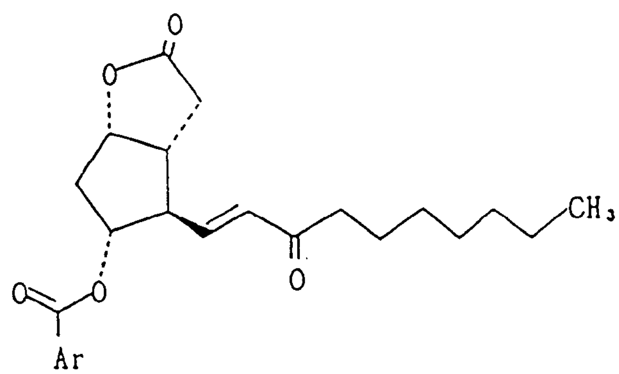

(3)

[wherein Ar represents an aromatic group]
which comprises subjecting a Corey lactone (1):

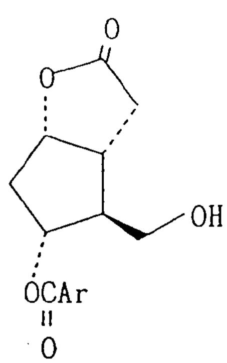

(1)

[wherein Ar is an aromatic group] to DMSO(dimethylsulfoxide) oxidation under the presence of a halogenated hydrocarbon with a dipole moment in the range of 0.9 D to 1.3 D to give a Corey aldehyde (2):

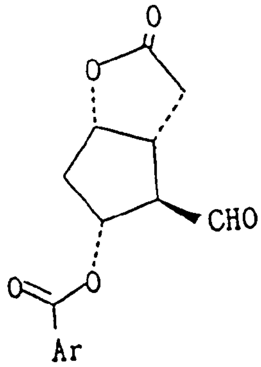

(2)

[wherein Ar is an aromatic group] and then reacting the obtained Corey aldehyde (2) with a di-lower alkyl(2-oxononyl)phosphonate:

$$(RO)_2POCH_2CO(CH_2)_6CH_3$$

[wherein R is a $C_1$ - $C_4$ alkyl group] under the presence of a lithium halide and a tertiary amine using the same halogenated hydrocarbon as is used in the DMSO oxidation as a solvent.

**5.** A preparation process according to Claim 4, wherein Ar is phenyl or biphenyl and the reaction solvent is dichloromethane.

**6.** A preparation process according to Claim 4, wherein the di-lower-alkyl (2-oxononyl)phosphonate is dimethyl (2-oxononyl)phosphonate.

**7.** A preparation process according to Claim 4, wherein DMSO oxidation is Pfilzner-Moffatt oxidation.